# EUROPEAN PATENT APPLICATION

(11) **EP 1 317 933 A1**
(43) Date of publication of application: **11.06.2003**
(21) Application number: 01965611.5
(22) Date of filing: 13.09.2001
(51) Int. Cl.: A61L 15/00, A61F 13/02, A61K 9/70, A61P 17/02

(54) **PREPARATIONS FOR COATING WOUND**

(30) Priority: 14.09.2000 JP 2000279363
(71) Applicant: SSP Co., Ltd., Chuo-ku, Tokyo 103-8481 (JP)
(72) Inventor: OTSUKA, Shigenori, CHIBA-SHI, Chiba 260-0832 (JP); MURATA, Misao, NARITA-SHI, Chiba 286-0045 (JP); UMEHARA, Norimitsu, TOKOROZAWA-SHI, Saitama 359-0025 (JP); MORIMOTO, Shinichi, NARITA-SHI, Chiba 286-0033 (JP); HANAOKA, Yoshiaki, YOTSUKAIDO-SHI, Chiba 284-0044 (JP); KASAI, Shuichi, NARITA-SHI, Chiba 286-0018 (JP); IMAMORI, Katsumi, YOTSUKAIDO-SHI, Chiba 284-0006 (JP)
(74) Representative: Wächtershäuser, Günter, Prof. Dr.
(86) International application number: JP0107945
(87) International publication number: WO02022182

(57) **Abstract**

Provided is an aqueous gel preparation for covering wounds which contains a paste having a gel strength, in water, of 7.5 to 100 g. Such a wound covering preparation is effective for promoting wound healing, is capable of absorbing exudate sufficiently, does not adhere to the wound surface and can be held at the affected part stably.

## Description

### Technical Field

The present invention relates to preparations for covering wounds capable of protecting and curing wounds conveniently.

### Background Art

Wound covering materials in various forms using alginic acid, chitin, hydrocolloid, polyurethane or the like have been put on the market, but they sometimes disturb wound healing by softening at the wound region, absorbing an exudate or by adhering to the wound surface. Although a hydrogel preparation is improved in these points, its weak adhesive force must be supplemented by the covering with an adhesive tape in order to hold the preparation at the wound region. This adhesive tape is accompanied with such problems that it sometimes causes a skin irritation or it must be changed frequently at the wound region rich in exudate because of its poor. water absorption property.

For example, in Japanese Patent Laid-Open No. Hei 5-84290, proposed is a hydrogel for covering wounds which uses a polymer having a temperature responsiveness. It has however such problems that after application of it to the wound surface, an adhesive tape or the like must be used to hold it to the affected part for protecting the applied surface and that it must be cooled when released therefrom. In Japanese Patent Laid-Open No. Hei 7-250886, proposed is a gel formed by the interaction between polyvinylpyrrolidone and chitosan. However, there are the problems that its adhesive power is not enough to hold long at the affected part, nor is it possible to absorb a sufficient amount of water.

### DISCLOSURE OF THE INVENTION

Accordingly, an object of the present invention is to provide a preparation for covering wounds which has an effect for promoting wound healing, exhibits a sufficient water absorbing property, thus can absorb a wound exudate, does not adhere to the wound surface and can be held stably at the affected part.

Under such situations, the present inventors have proceeded with an extensive investigation. As a result, it has been found that an aqueous gel type wound-covering preparation which has a paste exhibiting a gel strength in water within a predetermined range is excellent in an effect for promoting wound healing; and that the preparation having an adhesive force falling within a predetermined range upon saturation with water and at the normal time is equipped with both non-stickiness to the wound surface and holding property at the affected part, leading to the completion of the present invention.

The object of the present invention is to provide an aqueous gel type wound-covering preparation which has a paste exhibiting a gel strength in water ranging from 7.5 to 100 g.

### BRIEF DESCRIPTION OF THE DRAWINGS

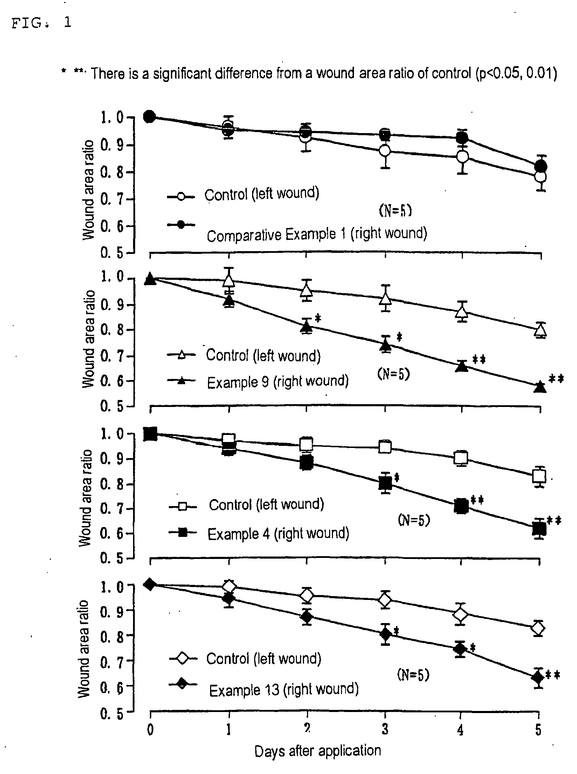

FIG. 1 illustrates an influence of the gel strength in water of a paste on the treatment of a punched wound made on the back of rats.

### BEST MODE FOR CARRYING OUT THE INVENTION

The preparation for covering wounds according to the present invention has a gel strength in water ranging from 7.5 to 100 g. The term "gel strength in water" as used herein means a stress applied to the paste at a press distance of 2 mm. It is measured by putting a cut piece of the preparation in a square petri dish, adding purified water of 10 times the weight of the paste to cause swelling for 24 hours, and measuring the stress by a rheometer (tension/stress tester). When the gel strength in water is less than 7.5 g, the preparation is inferior in an effect for promoting wound healing. When the gel strength in water exceeds 100 g, the preparation is too hard for the wound surface and at the same time, such a high gel strength is not necessary from the viewpoint of the effect for promoting wound healing. The gel strength in water of the preparation for covering wounds preferably ranges from 7.5 to 30 g, more preferably from 9 to 15 g.

The preparation for covering wounds according to the present invention preferably has a gel strength of 100 g or greater, more preferably from 100 to 300 g, at the normal time (when it is not swollen with water).

The paste of the preparation for covering wounds according to the present invention has a water swelling property. The term "water swelling property" as used herein means a property capable of absorbing water of at least 10 times the weight of the paste and expands its volume by water absorption. If its water absorption amount is below this level, it cannot absorb an exudate from the wound surface sufficiently and must be changed frequently.

To facilitate easy release from the wound surface without sticking thereto, it is preferred to lower the adhesive force of the paste of the invention preparation which has absorbed an exudate from the wound surface. More specifically, it has a ball tack less than 4 when it is saturated with water, with 3 or less being particularly preferred. From the viewpoint of the holding property at the affected part, it has a sufficient adhesive force necessary for adhesion to the normal skin, more specifically, it preferably has a ball tack of 4 or greater at the normal time (when not swelled with water). Excessively high adhesive force to the normal skin sometimes adversely affects the skin upon change of the preparation, so that the upper limit of the ball tack is preferably 15, with a ball tack ranging from 6 to 12 being more preferred. The term "adhesive force" as used herein means a value obtained in accordance with the adhesion test (usually called "ball tack") as described in the column of "Adhesion Test" on page 96 of "Iyakuhin Seizo Shishin 2000" Drug Preparation Guide 2000" (edited by Yakuji Shinsa Kenkyukai, published by Jihou Co., Ltd.).

The preparation for covering wounds according to the present invention can be prepared by adding, to an aqueous polymer, a gelling agent and a gelation regulator, an optional component such as filler, surfactant, oily component, humectant, water, and medicinal component.

Components use for its preparation will next be described.

### [Aqueous polymers]

An aqueous polymer is a polymer capable of forming a gel in the presence of water irrespective of how to form it. The aqueous polymer therefore takes part in the gel formation of a paste. Typical aqueous polymers include water-soluble polymers and hydrophilic-group-containing polymers. Specific examples include polyacrylic acid or salts thereof such as polyacrylic acid, sodium polyacrylate, crosslinked and branched polyacrylic acid, crosslinked and branched sodium polyacrylate, potassium polyacrylate, monoethanolamine polyacrylate, diethanolamine polyacrylate, triethanolamine polyacrylate and ammonium polyacrylate; copolymers having acrylic acid or salt thereof as a constituent such as N-vinylacetamide/sodium acrylate copolymer; cellulose derivatives or salts thereof such as hydroxyethyl cellulose, hydroxypropyl cellulose, hydroxypropyl methylcellulose, hydrophobic hydroxypropyl methylcellulose, methyl cellulose, carboxymethyl cellulose, and carboxymethylcellulose sodium; and polyvinyl alcohol, polyvinylpyrrolidone, polyethylene oxide, methyl vinyl ether/maleic anhydride copolymer, polyacrylamide, alginic acid, sodium alginate, propyleneglycol alginate, gum arabic, tragacanth gum, locust bean gum, guar gum, tamarind gum, xanthan gum, Gellan gum, carrageenan and agar. Out of them, polyacrylic acid, sodium polyacrylate, crosslinked and branched polyacrylic acid, crosslinked and branched sodium polyacrylate, crosslinked graft copolymer of acrylic acid/starch, N-vinylacetamide/sodium acrylate copolymer, hydroxyethyl cellulose, hydroxypropyl cellulose, hydroxypropylmethyl cellulose, carboxymethyl cellulose and carboxymethylcellulose sodium are preferred, with polyacrylic acid, sodium polyacrylate and carboxymethylcellulose sodium being particularly preferred. These aqueous polymers may be used either singly or in combination. They are usually added in an amount of 0.5 to 50 wt.%, preferably from 2 to 40 wt.%, more preferably from 3 to 30 wt.%, especially from 3 to 10 wt. %, based on the total weight of the paste.

### [Gelling agent]

No particular limitation is imposed on the gelling agent insofar as it causes gelation by a physicochemical method employed depending on the properties of the aqueous polymer employed. For example, when a paste is prepared by the crosslinking reaction of the aqueous polymer, any gelling agent is usable insofar as it enables crosslinking of the aqueous polymer, but polyvalent metal compounds are preferred. As the polyvalent metal compounds, aluminum compounds, magnesium compounds and calcium compounds are particularly preferred. Specific examples include aluminum potassium sulfate, aluminum ammonium sulfate, aluminum hydroxide, aluminum sulfate, aluminum chloride, aluminum glycinate, acetoglutamide aluminum, aluminum acetate, aluminum oxide, synthetic aluminum silicate, aluminum metasilicate, calcium hydroxide, calcium carbonate, calcium sulfate, calcium nitrate, calcium chloride, calcium acetate, calcium oxide, calcium phosphate, magnesium hydroxide, magnesium carbonate, magnesium sulfate, magnesium acetate, magnesium silicate, magnesium oxide, alumina-magnesium hydroxide, magnesium aluminate metasilicate, magnesium aluminate silicate and synthetic hydrotalcite, and hydrates or anhydrides thereof. These gelling agents may be used either singly or in combination. They are usually added in an amount of from 0.001 to 10 wt.%, preferably from 0.01 to 5 wt.%, more preferably from 0.05 to 3 wt. %, especially from 0.1 to 2 wt.%, based on the total weight of the paste.

### [Gelation regulator]

A gelation regulator can be added to control the preparation ease and physical properties such as softness of the gel. Examples of the gelation regulator include chelating agents such as sodium EDTA and sodium metaphosphate; organic acids such as lactic acid, citric acid and tartaric acid, or metal salts thereof; inorganic acids such as sulfuric acid and hydrochloric acid; organic bases such as diethylamine, diethanolamine, triethanolamine, and diisopropanolamine; and inorganic bases such as sodium hydroxide and ammonia. These gelation regulators may be used either singly or in combination. Their amount differs, depending on the nature of the gelation regulator, but they are usually added in an amount of from 0.001 to 10 wt.%, preferably from 0.01 to .5 wt.%, more preferably from 0.05 to 2 wt.%, especially from 0.1 to 2 wt.%, based on the total amount of the paste.

Examples of a preferred combination of the aqueous polymer, gelling agent and gelation regulator for actualizing the above-described gel strength in water and adhesive force include a combination of polyacrylic acid or salt thereof (especially, having a molecular weight of from 3000'to 10000000 and a polymerization degree of 40 to 100000) and carboxymethylcellulose sodium (especially, having a molecular weight of 8000. to 300000 and a polymerization degree of 40 to 1500) as the aqueous polymer, an aluminum compound as the gelling agent and a chelating agent as the gelation regulator; and a combination of partially neutralized polyacrylic acid and carboxymethylcellulose sodium as the aqueous polymer, burnt alum (anhydride of aluminum potassium sulfate) as the gelling agent and sodium EDTA as the gelation regulator. The gelling agent and gelation regulator are preferably mixed at a molar ratio ranging from 1:1 to 1:3, with a range of from 1:1.1 to 1:2.5 being more preferred.

### [Filler]

A filler can be added to keep formability and strength of the paste. No particular limitation is imposed on its kind and either inorganic or organic filler may be added. Examples include kaolin, titanium oxide, bentonite, light silicic anhydride, hydrophobic light silicic anhydride and starch acrylate. These fillers may be used either singly or in combination. They are preferably added in an amount of from 0.01 to 30 wt.%, more preferably from 0.02 to 20 wt.%, especially from 0.03 to 15 wt.%, based on the total weight of the paste.

### [Surfactant]

A surfactant can be added to facilitate addition of an oily component or medicinal component. Ionic and nonionic surfactants may be used. Examples include alkylallyl polyether alcohols, higher alcohol sulfates, N-cocoyl-L-arginine ethyl ester, DL-pyrrolidone carboxylate, sodium N-cocoyl-N-methylaminoethylsulfonate, cholesterol, self-emulsified glycerin monostearate, sucrose fatty acid esters, squalane, stearyl alcohol, polyoxyl (40) stearate, sorbitan sesquioleate, cetanol, cetomacrogol 1000, diethyl sebacate, sorbitan fatty acid esters, sodium dodecylbenzenesulfonate, sorbitan trioleate, nonylphenoxypolyoxyethylene ethane sulfate ammonium, polyoxyethylene octylphenyl ether, polyoxyethylene oleylamine, polyoxyethylene hydrogenated castor oil 20, polyoxyethylene. hydrogenated castor oil 60, polyoxyethylene stearyl ether, polyoxyethylene cetyl ether, polyoxyethylene sorbitan monolaurate, polyoxyethylene sorbit beeswax, polyoxyethylene nonylphenyl ether, polyoxyethylene (105) polyoxypropylene (5) glycol, polyoxyethylene (120) polyoxypropylene (40) glycol, polyoxyethylene (160) polyoxypropylene (30) glycol, polyoxyethylene (20) polyoxypropylene (20) glycol, polyoxyl 35 castor oil, polysorbate 20, polysorbate 60, polysorbate 80, macrogol 400, sorbitan monooleate, glycerin monostearate, sorbitan monostearate, sorbitan monolaurate, DL-pyrrolidone carboxylic acid salt of N-coconut oil fatty acid acyl-L-arginine ethyl, lauryl dimethylamine oxide solution, sodium laurylsulfate, lauric acid diethanolamide, lauroyl sarcosine sodium, lauromacrogol, sodium phosphate polyoxyethylene lauryl ether, and phosphoric acid polyoxyethylene (8) oleyl ether. These surfactants may be used either singly or in combination. They are preferably added in an amount of from 0.001 to 30 wt.%, more preferably from 0.02 to 20 wt.%, especially from 0.03 to 10 wt.%, based on the total weight of the paste.

### [Oily component]

The oily component takes part in the releasability from a release film, release paper or affected part and it is added as needed. Examples include plant oils or fats such as olive oil, sesame oil, soybean oil, tsubaki oil, rapeseed oil, castor oil, coconut oil and peanut oil, animal oils or fats such as yolk oil and mink oil; waxes such as beeswax, whale wax, purified lanolin and carnauba wax, hydrocarbons such as liquid paraffin, squalane, paraffin wax and vaseline, natural or synthetic fatty acids such as oleic acid, lauric acid, myristic acid, stearic acid and isostearic acid; natural or synthetic higher alcohols such as cetanol, stearyl alcohol, hexyl decanol, octyl dodecanol and lauryl alcohol; and esters such as diisopropyl adipate, isopropyl myristate, isopropyl palmitate, octyldodecyl myristate, octyldodecyl oleate, diisopropyl sebacate and diethyl sebacate. These oily components may be used either singly or in combination. They are preferably added in an amount of from 0.01 to 30 wt.%, more preferably from 0.02 to 15 wt.%, especially from 0.03 to 10 wt.%, based on the total weight of the paste.

### [Humectant]

The humectant can be added to avoid the affected surface from drying. Polyhydric alcohols are typical examples of the humectant, but the other substances having moisture retention capacity can be added without particular limitation. Examples include glycerin, concentrated glycerin, sorbitol, sorbitol solution, propylene glycol, polyethylene glycol and urea. These humectants may be added either singly or in combination. They are preferably added in an amount of from 1 to 95 wt.%, more preferably from 2 to 80 wt.%, especially from 3 to 60 wt.%, based on the total weight of the paste.

### [Water]

Water is usually added in an amount of from 0 to 80 wt.%, preferably from 10 to 70 wt.%, more preferably from 20 to 60 wt.%, especially preferably from 40 to 50 wt.% based on the total weight of the paste.

### [Medicinal component]

Although no particular limitation is imposed on the medicinal component insofar as it can be applied to the wound region, the below-described ones are usable. They may be used either singly or in combination, as needed.

### (Bactericide)

Examples of the bactericide include acrinol, benzalkonium chloride, benzethonium chloride, chlorhexidine gluconate, iodine, iodine tincture, iodoform, and povidone iodine.

### (Styptic)

Examples of the styptic include thrombin, sodium alginate, ε-aminocaproic acid, monoethanolamine oleate, carbazochrome sodium sulfonate and tranexamic acid.

### (Opioid analgesic)

Examples of the opioid analgesic include morphine hydrochloride and morphine sulfate.

### (Sulfa drug)

Examples of the sulfa drug include salazosulfapyridine, sulfadiazine, sulfadiazine silver, sulfadimethoxine, sulfamethizole, sulfamethoxazole, sulfamonomethoxine, sulfisomidine and sulfisomidine sodium.

### (Antibiotic)

Examples of the antibiotic include vancomycin hydrochloride, lincomycin hydrochloride, clindamycin, teicoplanin, phenethicillin potassium, benzylpenicillin potassium, benzylpenicillin benzathine, mupirocin calcium hydrate, arbekacin sulfate, aztreonam, spectinomycin hydrochloride, pivmecillinam hydrochloride, carumonam sodium, colistin sodium methanesulfonate, cefsulodin sodium, ceftibuten, tobramycin, amikacin sulfate, isepamicin sulfate, kanamycin sulfate, fradiomycin sulfate, polymixin B sulfate, aspoxicillin, amoxicillin, ampicillin, ampicillin sodium, cefetamet pivoxil hydrochloride, cefepime dihydrochloride, cefozopran hydrochloride, cefotiam hydrochloride, cefotiam hexetil hydrochloride, cefcapene pivoxil hydrochloride, cefmenoxime hydrochloride, talampicillin hydrochloride, bacampicillin hydrochloride, lenampicillin hydrochloride, ciclacillin, sulbenicillin sodium, cefaclor, cefazolin sodium, cefatrizine propylene glycol, cefadroxil, cefapirin sodium, cefamandole sodium, cefalexin, cefalotin sodium, cefaloridine, cefixime, cefoxitin sodium, ceftazidime sodium, cefotaxime sodium, cefotetan sodium, cefoperazone sodium, cefditoren pivoxil, cefdinir, ceftazidime, ceftizoxime sodium, ceftezole sodium, cefteram pivoxil, ceftriaxone sodium, cefpiramide sodium, cefbuperazone sodium, cefpodoxime proxetil, cefminox sodium, cefmetazole sodium, cefradine, cefroxadine, cefuroxime axetil, cefuroxime sodium, ticarcillin sodium, sultamicillin tosilate, piperacillin sodium, faropenem sodium, flomoxef sodium, fosfomycin, meropenem trihydrate, latamoxef sodium, astromicin sulfate, gentamicin sulfate, sisomycin sulfate, dibekacin sulfate, cefoselis sulfate, cefpirome sulfate, netilmicin sulfate, bekanamycin sulfate, micronomicin sulfate, ribostamycin sulfate, acetylkitasamycin, acetylspiramycin, erythromycin ethylsuccinate, erythromycin, erythromycin estolate, kitasamycin, clarithromycin, midecamycin acetate, kitasamycin tartrate, josamycin, erythromycin stearate, josamycin propionate, midecamycin, erythromycin lactobionate, roxithromycin, rokitamycin, tetracycline hydrochloride, demethylchlortetracycline hydrochloride, doxycycline hydrochloride, minocycline hydrochloride, chloramphenicol, chloramphenicol sodium succinate, chloramphenicol palmitate, cycloserine, rifampicin, enviomycin sulfate, streptomycin sulfate, oxytetracycline hydrochloride, gramicidin hydrochloride S, tetracycline, nadifloxacin, bacitracin, sodium fusidate, and colistin sulfate.

### [Dosage form]

The dosage form of the preparation for covering wounds according to the present invention is preferably a sheet, more typically, a sheet obtained by spreading a paste on a backing material. No particular limitation is imposed on the backing material insofar as it is a woven cloth, nonwoven cloth, film or sheet having flexibility. Examples include a woven fabric or nonwoven fabric obtained from fibers such as rayon, polyester, polyolefin and urethane, a polymer film and foamed sheet. The backing material preferably has stretchability in every direction. It may be subjected to anchor coating as needed. Instead of such a sheet obtained by spreading a paste on a backing material, that having a protective film on each side is usable.

### [Preparation process]

Although there is no particular limitation imposed on the preparation process of the preparation for covering wounds according to the present invention, when it is spread on a backing material, examples of the process include a process of preparing a paste by mixing the above-described components, spreading the mixture on the backing sheet and then covering the surface with a protective film; and a process of spreading the paste on a protective film, covering the film surface with a backing material and then transferring the paste to the backing material. The base material may be subjected to anchor coating as needed. When the preparation is in the dosage form free of a backing material, on the other hand, examples of the process include a process of spreading the paste on a protective film and then covering the paste surface further with a protective film, and a process of putting a paste in a mold of a predetermined size to form a sheet.

When the paste and/or backing material to be used for the preparation for covering wounds according to the present invention does not contain a bactericide, it is preferably subjected to sterilizing treatment. No particular limitation is imposed on the sterilization method, but examples of it include γ-radiation sterilization, electron beam sterilization, high-pressure steam sterilization, and ethylene oxide sterilization, of which γ-radiation sterilization, ethylene oxide sterilization, and high-pressure sterilization are preferred, with γ-radiation sterilization and high-pressure sterilization, particularly γ-radiation sterilization being more preferred.

Components derived from animals such as gelatin are preferably not added in order to avoid the risk of the wound-covering preparation being infected by bovine spongiform encephalopathy via a base.

The preparation for covering wounds according to the present invention thus prepared is preserved in an airtight container as needed.

### Examples

The present invention will hereinafter be described in further detail by Examples, but it is not limited to or by them. In Examples and Comparative Examples, the gel strength and adhesive force were measured in the below-described manners.

### (Gel strength in water and gel strength at the normal time)

A test substance is cut into a piece of 2.5 cm × 5 cm or 5 cm × 5 cm and placed in a square petri dish. Purified water is added in an amount of 10 times the weight of a paste to cause its swelling for 24 hours. A stress at a press distance of 2 mm is measured by a rheometer (tension/stress tester) as a gel strength in water. The gel strength at the normal time is measured in a similar manner by the rheometer (tension/stress tester) after putting the paste in a cylindrical container.

### (Adhesive force upon saturation with water and that at the normal time)

A test substance is cut into a piece of 5 cm × 5 cm. In a petri dish filled with purified water in an enough amount to immerse the test substance therewith, the test substance is immersed. It is allowed to stand for 1 hour to saturate it with water. The test substance saturated with water and the test substance before swelling with water are tested in accordance with an adhesion test (usually called "ball tack") described as a reference in the column of "Adhesion test" on page 96 of "Iyakuhin Seizo Shishin 2000" (edited by Yakuji Shinsa Kenkyukai, published by Jihou Co., Ltd.) and the ball number of the largest ball that has stopped is designated as a ball tack number.

### Examples 1 to 25 and Comparative Examples 1 to 3

In accordance with the formulations as shown in Tables 1 to 4, components were mixed to prepare a paste. The resulting paste was spread on a nonwoven fabric (polyester) and its surface was covered with a protective film to yield a preparation for covering wounds according to the present invention. The wound covering preparations thus obtained in Examples 4, 9, and 13 and Comparative Example 1 were each filled in an aluminum-made bag, followed by sterilization with γ-radiation.

**Table 1**

| | Examples | | | | | | | |
|---|---|---|---|---|---|---|---|---|
| Component (g) | 1 | 2 | 3 | 4 | 5 | 6 | 7 | 8 |
| Acrinol | | | | | | | | |
| Benzalkonium chloride | | | | | | | | |
| D-sorbitol solution (70%) | | 25 | 25 | | | | | |
| Polyvinyl alcohol *1 | | | | | | | | |
| Sodium EDTA | 0.14 | 0.21 | 0.27 | 0.28 | 0.32 | 0.36 | 0.4 | 0.44 |
| Tartaric acid | 1.2 | 1.2 | 1.2 | 1.2 | 1.2 | 1.2 | 1.2 | 1.2 |
| Lactic acid | | | | | | | | |
| Castor oil | 1 | 1 | 1 | 1 | 1 | 1 | 1 | 1 |
| Liquid paraffin | | | | | | | | |
| Propylene glycol | | | | | | | | |
| Polysorbate 80 | 0.1 | 0.1 | 0.1 | 0.1 | 0.1 | 0.1 | 0.1 | 0.1 |
| Polyethylene glycol monolaurate | | | | | | | | |
| Concentrated glycerin | 43 | 18 | 18 | 43 | 43 | 43 | 43 | 43 |
| Partially neutralized polyacrylic acid *2 | 5 | 6 | 6 | 5 | 5 | 5 | 5 | 5 |
| Aqueous solution of polyacrylic acid (20%) *3 | | | | | | | | |
| Sodium polyacrylate *4 | | | | | | | | |
| Carboxymethylcellulose sodium *5 | 2 | 3.5 | 3.5 | 2 | 2 | 2 | 2 | 2 |
| Kaolin | | | | | | | | |
| Titanium oxide | | | | | | | | |
| Burnt alum | 0.18 | 0.27 | 0.27 | 0.36 | 0.36 | 0.36 | 0.36 | 0.36 |
| Aluminum glycinate | | | | | | | | |
| Tricalcium phosphate | | | | | | | | |
| Purified water | 47.38 | 44.72 | 44.66 | 47.06 | 47.02 | 46.98 | 46.98 | 46.98 |
| Total | 100 | 100 | 100 | 100 | 100 | 100 | 100 | 100 |
| Gel strength in water (g) | 21.2 | 20.3 | 15.7 | 11.0 | 11.3 | 12.0 | 11.1 | 7.7 |
| Gel strength at the normal time (g) | 183.2 | 174.3 | 126.8 | 204.9 | 154.7 | 138.0 | 150.6 | 120.3 |
| Adhesive force upon saturation with water | No 3 or less | | | | | | | |
| Adhesive force at the normal time | No.10 | No, 10 | No.11 | No.9 | No.10 | No.10 | No.11 | No. 11 |

**Table 2**

| | Examples | | | | | | |
|---|---|---|---|---|---|---|---|
| Component (9) | 9 | 10 | 11 | 12 | 13 | 14 | 15 |
| Acrinol | | | | | | 0.5 | |
| Benzalkonium chloride | | | | | | | 0.01 |
| D-sorbitol solution (70%) | 25 | 25 | 25 | 25 | 25 | | |
| Polyvinyl alcohol *1 | | 0.2 | | | | | 0.2 |
| Sodium EDTA | 0.32 | 0.21 | 0.2 | 0.21 | 0.21 | 0.28 | 0.28 |
| Tartaric acid | 1.2 | 1.2 | 1.2 | 1.2 | 1:2 | 1.2 | 1.2 |
| Lactic acid | | | | | | | |
| Castor oil | 1 | 1 | 1 | 1 | 1 | 1 | 1 |
| Liquid paraffin | | | | | | | |
| Propylene glycol | | 1 | | | | | |
| Polysorbate 80 | 0.1 | 0.1 | 0.1 | 0.1 | 0.1 | 0.1 | 0.1 |
| Polyethylene glycol monolaurate | | | | | | | |
| Concentrated glycerin | 18 | 18 | 18 | 18 | 18 | 43 | 43 |
| Partially neutralized polyacrylic acid *2 | 6 | 6 | 6 | 6 | 6 | 5 | 5 |
| Aqueous solution of polyacrylic acid (20%) *3 | | | | | | | |
| Sodium polyacrylate *4 | | | | | | | |
| Carboxymethylcellulose sodium ^{*}5 | 3.5 | 3.5 | 3.5 | 3.5 | 3.5 | 2 | 2 |
| Kaolin | | 0.5 | 0.5 | | 0.5 | | 0.5 |
| Titanium oxide | | 0.2 | 0.2 | | 0.2 | 0.5 | |
| Burnt alum | 0.27 | 0.27 | | 0.3 | 0.33 | 0.36 | 0.36 |
| Aluminum glycinate | | | 0.2 | | | | |
| Tricalcium phosphate | | | | | | | |
| Purified water | 44.61 | 42.62 | 43.90 | 44.69 | 43.96 | 46.56 | 46.36 |
| Total | 100 | 100 | 100 | 100 | 100 | 100 | 100 |
| Gel strength in water (g) | 7.5 | 14.4 | 13.6 | 26.0 | 30.0 | 10.6 | 13.8 |
| Gel strength at the normal time (g) | 102.4 | 169.2 | 150.6 | 199.6 | 226.8 | 124.6 | 145.7 |
| Adhesive force upon saturation with water | No 3 or less | | | | | | |
| Adhesive force at the normal time | No.10 | No.10 | No.11 | No.10 | No.7 | No.9 | No.10 |

**Table 3**

| | Examples | | | | | | |
|---|---|---|---|---|---|---|---|
| Component (g) | 16 | 17 | 18 | 19 | 20 | 21 | 22 |
| Acrinol | | | | | | | |
| Benzalkonium chloride | | | | | | | |
| D-sorbitol solution (70%) | | | | | | | 20 |
| Polyvinyl alcohol *1 | | | | | | | |
| Sodium EDTA | 0.05 | 0.5 | 0.05 | 0.5 | 0.05 | 0.5 | 0.2 |
| Tartaric acid | 1.2 | 1.2 | 0.8 | 0.8 | 1.8 | 1.8 | |
| Lactic acid | | | | | | | 1.2 |
| Castor oil | 2 | 1 | | 1 | 0.5 | 1 | 1 |
| Liquid paraffin | | | 1 | | 0.5 | | |
| Propylene glycol | | | | | | | |
| Polysorbate 80 | 0.1 | 0.1 | | 0.1 1 | 0.1 | 0.1 | 0.1 |
| Polyethylene glycol monolaurate | | | 0.1 | | | 0.1 | |
| Concentrated glycerin | 43 | 43 | 43 | 43 | 43 | 43 | 25 |
| Partially neutralized polyacrylic acid *2 | 8 8 | 2 2 | | | | | 3 3 |
| Aqueous solution of polyacrylic acid (20%) *3 | | | 40 40 | 10 10 | | | |
| Sodium polyacrylate *4 | | | | | 8 | 2 | |
| Carboxymethylcellulose sodium *5 | 1 | 3.5 | 1 | 3.5 | 1 | 3.5 | 3.5 |
| Kaolin | 0.5 | | 0.5 | | 0.4 | | 3 |
| Titanium oxide | 0.2 | | 0.1 | | 0.2 | | 0.5 |
| Burnt alum | 0.05 | 0.5 | 0.05 | 0.5 | 0.05 | 0.5 | 0.2 |
| Aluminum glycinate | | | | | | | |
| Tricalcium phosphate | | | | | | | |
| Purified water | 43.9 | 48.2 | 13.4 | 40.6 | 44.4 | 47.5 | 42.3 |
| Total | 100 | 100 | 100 | 100 | 100 | 100 | 100 |
| Gel strength in water (g) | 38.1 | 18.4 | 7.9 | 16.8 | 49.7 | 19.3 | 12.4 |
| Gel strength at the normal time (g) | 118.1 | 169.4 | 167.5 | 230.5 | 197.4 | 156.3 | 174.9 |
| Adhesive force upon saturation with water | No 3 or less | | | | | | |
| Adhesive force at the normal time | No.10 | No. 8 | No.11 | No.9 | No. 10 | No. 7 | No. 8 |

**Table 4**

| | Examples | | | Comparative Examples | | |
|---|---|---|---|---|---|---|
| Component (g) | 23 | 24 | 25 | 1 | 2 | 3 |
| Acrinol | | | | | | |
| Benzalkonium chloride | | | | | | |
| D-sorbitol solution (70%) | 20 | 15 | 15 | 25 | | 25 |
| Polyvinyl alcohol *1 | | | 2 | | | |
| Sodium EDTA | 2 | 2 | 3 | 0.12 | 0.48 | 0.12 |
| Tartaric acid | 0.8 | 1 | | 1.2 | 1.2 | 1.2 |
| Lactic acid | | | 1.5 | 1 | 1 | 1 |
| Castor oil | 1 | 1 | | | | |
| Liquid paraffin | | | | | | |
| Propylene glycol | | | | | | |
| Polysorbate 80 | 0.1 | 0.1 | | 0.1 | 0.1 | 0.1 |
| Polyethylene glycol monolaurate | | 0.1 | | | | |
| Concentrated glycerin | 30 | 25 | 20 | 18 | 43 | 18 |
| Partially neutralized polyacrylic acid *2 | 2.5 | 2.5 | | 6 6 | 5 5 | 6 6 |
| Aqueous solution of polyacrylic acid (20%) *3 | | | | | | |
| Sodium polyacrylate *4 | | 1.5 | 7 | | | |
| Carboxymethylcellulose sodium *5 | 0.5 | 0.5 | 2 | 3.5 | 2 | 3.5 |
| Kaolin | | 2 | | | | 2 |
| Titanium oxide | | | | | | |
| Burnt alum | 2 | 2 | | 0.09 | 0.36 | 0.09 |
| Aluminum glycinate | | | | | | |
| Tricalcium phosphate | | | 3 | | | |
| Purified water | 41.1 | 49.8 | 46.5 | 44.99 | 46.98 | 42.99 |
| Total | 100 | 100 | 100 | 100 | 100 | 100 |
| Gel strength in water (g) | 69.6 | 76.9 | 13.6 | 2.2 | 1.9 | 1.0 |
| Gel strength at the normal time (g) | 215.6 | 281.4 | 142.0 | 97.2 | 150.9 | 109.0 |
| Adhesive force upon saturation with water | No. 3 or less | | | | | |
| Adhesive farce at the normal time | No.8 | No.7 | No.11 | No.10 | No.10 | No.10 |
| *1: average molecular weight: 20000 to 30000, average polymerization degree: 400 to 600 | | | | | | |
| *2: average molecular weight: 3500000 to 5000000, average polymerization degree: 40000 to 60000 | | | | | | |
| *3: average molecular weight: 220000 to 360000, average polymerization degree: 3000 to 5000 | | | | | | |
| *4: average molecular weight: 2300000 to 4800000, average polymerization degree: 25000 to 50000 | | | | | | |
| *5: average molecular weight: 100000 to 200000, average polymerization degree: 500 to 1000 | | | | | | |

### Example 26

A methylvinyl ether/maleic anhydride copolymer (average molecular weight: 20000 to 70000, polymerization degree; 130 to 450) and concentrated glycerin were mixed at a weight ratio of 1:1 to dissolve the former in the latter. The resulting solution was charged in a mold of a predetermined size and preserved at 100°C for 100 minutes to obtain a sheet-like gel substance (gel strength in water: 54.1 g, gel strength at the normal time: 204.7 g, ball tack when saturated with water: No. 3 or less, ball tack at the normal time: No. 6).

### Example 27

A 10% aqueous solution of a methylvinyl ether/maleic anhydride copolymer (average molecular weight: 20000 to 70000, polymerization degree; 130 to 450) and a 10% aqueos solution of PVP (average molecular weight: 44000 to 54000, polymerization degree: 396 to 486) were mixed at a weight ratio of 1:1. The resulting solution was charged in a mold of a predetermined size and preserved at 100°C for 100 minutes to obtain a sheet-like gel substance (gel strength in water: 61.8 g, gel strength at the normal time: 221.7 g, ball tack when saturated with water: No. 3 or less, ball tack at the normal time: No. 7).

### Example 28

(A) In 30 g of concentrated glycerin were dispersed 5 g of a methylvinyl ether/maleic anhydride copolymer (average molecular weight: 20000 to 70000, polymerization degree; 130 to 450) and 5 g of sodium polyacrylate (2300000 to 4800000, polymerization: 25000 to 50000). (B) In 15 g of concentrated glycerin was dispersed 1 g of a crosslinking agent. (C) In 25 g of purified water were dissolved and mixed 15 g of D-sorbitol solution and 4 g of kaolin. (A), (B) and (C) were kneaded uniformly and the resulting mass was spread on a nonwoven fabric, whereby a sheet-like gel substance (gel strength in water: 19.3 g, gel strength at the normal time: 152.9 g, ball tack when saturated with water: No. 3 or less, ball tack at the normal time: No. 11) was obtained.

### Example 29

Mixed were 10 g of sodium polyacrylate (2300000 to 4800000, polymerization degree; 25000 to 50000), 30 g of carboxymethylcellulose sodium (molecular weight: 100000 to 200000, polymerization: 500 to 1000), 0.05 g of sodium EDTA and 54.9 g of purified water. To the resulting mixture was added a dispersion of 0.05 g of burnt alum in 5 g of propylene glycol, followed by further mixing. The resulting mixture was charged in a mold of a predetermined size, and preserved at 100°C for 100 minutes to obtain a sheet-like gel substance (gel strength in water: 14.5 g, gel strength at the normal time: 201.8 g, ball tack when saturated with water: No. 3 or less, ball tack at the normal time: No. 8).

### Example 30

Mixed were 5 g of sodium polyacrylate (2300000 to 4800000, polymerization degree; 25000 to 50000), 45 g of carboxymethylcellulose sodium (molecular weight: 100000 to 200000, polymerization degree: 500 to 1000), 0.05 g of sodium EDTA and 44.9 g of purified water. To the resulting mixture was added a dispersion of 0.05 g of burnt alum in 5 g of propylene glycol, followed by further mixing. The resulting mixture was charged in a mold of a predetermined size, and preserved at 100°C for 100 minutes to obtain a sheet-like gel substance (gel strength in water: 18.7 g, gel strength at the normal time: 137.4 g, ball tack when saturated with water: No. 3 or less, ball tack at the normal time: No. 8).

### Test 1 (Influence of gel strength on the treatment of punched-out wound on the rat back skin)

On the back of each of twenty rats, a wound was made symmetrically by punching out its dorsal skin tissue under anesthesia with ether. They were classified into four groups. A control (base cloth, 12 mm × 12 mm) and a test preparation (Example 4, Example 9, Example 13 or Comparative Example 1, each 12 mm × 12 mm) were applied sterilely on the left and right wound surfaces, respectively once a day for 5 days and the area (longer diameter of the wound × shorter diameter of the wound) of the punched-out wound was measured. The results are shown in FIG. 1.

As is apparent from the results shown in FIG. 1, the preparations of Examples 4, 9 and 13 each exhibited a significant decrease in the area ratio compared with that of the control group, suggesting they had a wound healing promoting effect.

### Test 2 (water absorption test)

Water absorbing property was compared between the invention product and commercially available wound covering preparations.

A petri dish having purified water poured therein is weighed. A test substance is then dipped in the water. The dish is then covered and the test substance is allowed to stand still. After an elapse of sufficient time, the test substance is taken out from the petri dish and the dish is weighed. A difference in the weight of the petri dish is calculated as a water absorption amount. The results are shown in Table 5.

**Table 5**

| Water absorption amount (g) | | | | | | | | | |
|---|---|---|---|---|---|---|---|---|---|
| | Hour (hr) | | | | | | | | |
| | 0 | 0.25 | 0.5 | 1 | 1.5 | 2 | 4 | 16 | 24 |
| Example 4 | 0 | 2.38 | 3.24 | 4.03 | 4.29 | 4.54 | 4.75 | 4.76 | 4.77 |
| Commercial preparation A | 0 | 0.16 | 0.21 | 0.25 | 0.26 | 0.43 | 0.50 | 1.30 | 1.87 |
| Commercial preparation B | 0 | 0.65 | 0.90 | 1.22 | 1.46 | 1.71 | 1.89 | 2.20 | 2.32 |
| Commercial preparation C | 0 | 0.61 | 0.84 | 1.13 | 1.51 | 1.85 | 2.25 | 2.51 | 2.77 |
| Commercial preparation D | 0 | 0.81 | 1.04 | 1.47 | 1.81 | 2.03 | 2.67 | 3.77 | 4.25 |
| Commercial preparation E | 0 | 007 | 0.10 | 0.20 | 0.30 | 0.37 | 0.87 | 1.83 | 2.77 |
| Commercial preparation F | 0 | 0.27 | 0.33 | 0.43 | 0.53 | 0.63 | 0.90 | 1.24 | 1.57 |

Commercial preparation A: hydrophobic wound covering preparation [Hydrocolloid (carboxymethylcellulose sodium, pectin, gelatin)]
Commercial preparation B: Water-based wound covering preparation [Hydrogel (polyacrylamide, agar)]
Commercial preparation C: Water-based wound covering preparation [Hydrogel (polyacrylamide, agar)]
Commercial preparation D: Water-based wound covering preparation [Hydrogel (polyvinylpyrrolidone)]
Commercial preparation E: Oily wound covering preparation [Hydrocolloid (carboxymethylcellulose sodium)]
Commercial preparation F: Oily wound covering preparation [Hydrocolloid (carboxymethylcellulose sodium)]

The invention product (Example 4) is presumed to be prompt in absorbing an exudate and maintaining the wound surface to be appropriately wet. Even upon release after use, it has little stimulation to the wound surface, suggesting that it is not harmful to the skin.

### Test 3 (Clinical test)

To the wound region of 15 subjects, the wound covering preparation (10 cm × 14 cm, weight of the paste: about 14 g) of Example 4 was applied once or twice a day for 4 weeks in principle. If the wound was cured within these four weeks, the test was brought to completion at the this time. The effectiveness of the preparation compared with the state at the starting time of the test was judged. The results are shown in Table 6.

The effectiveness was judged on four criteria, that is, markedly effective, effective, slightly effective, ineffective, comprehensively from the degree of improvement based on synthetic skin findings (amount of secretion, granulation, formation of the epidermis, pain, reddening around the applied part), a contraction ratio of the ulcer (wound) area, and convenience observed through the test term (adhesion to the wound part, penetration of a secretion through a nonwoven fabric, pain upon change of the preparation, gel residue on the wound).

**Table 6**

| Markedly effective | Effective | Slightly effective | Ineffective | Total |
|---|---|---|---|---|
| 8 subjects (53%) | 4 subjects (27%) | 1 subject (7%) | 2 subjects (13%) | 15 subjects |

### Industrial Applicability

The preparation for covering wounds according to the present invention has excellent effects for promoting healing of wounds and is equipped with both non-stickiness to the wound surface and retention to the affected part.

## Claims

1. An aqueous gel preparation for covering wounds, comprising a. paste having a gel strength in water of from 7.5 to 100 g.

2. An aqueous gel preparation for covering wounds, comprising a paste having a gel strength in water of from 7.5 to 30 g.

3. An aqueous gel preparation for covering wounds according to Claim 1 or 2, wherein the paste swells with water.

4. An aqueous gel preparation for covering wounds according to any one of Claims 1 to 3, wherein the paste saturated with water has an adhesive force less than 4 in terms of ball tack.

5. An aqueous gel preparation for covering wounds according to Claim 4, wherein the paste has an adhesive force of 4 or greater in terms of ball tack.

6. An aqueous gel preparation for covering wounds according to Claim 4, wherein the paste has an adhesive force of 4 to 15 in terms of ball tack.

7. An aqueous gel preparation for covering wounds according to any one of Claims 1 to 6, wherein the paste has a gel strength, at the normal time, of 100 g or greater.

8. An aqueous gel preparation for covering wounds according to any one of Claims 1 to 7, which is in the sheet form.

9. An aqueous gel preparation for covering wounds according to Claim 8, which is obtained by spreading the paste on a backing material.

10. An aqueous gel preparation for covering wounds according to Claim 9, wherein the backing material is a nonwoven fabric, fabric or film.

11. An aqueous gel preparation for covering wounds according to any one of Claims 1 to 10, wherein the paste and/or backing material has been sterilized.

12. An aqueous gel preparation for covering wounds according to Claim 11, wherein the sterilization is effected by γ-radiation.

13. An aqueous gel preparation for covering wounds according to Claim 1, wherein the paste contains an aqueous polymer, a gelling agent and a gelation regulator.
